# EUROPÄISCHE PATENTSCHRIFT

(11) **EP 0 699 731 B1**
(45) Veröffentlichungstag und Bekanntmachung des Hinweises auf die Patenterteilung: **04.10.2000**
(21) Anmeldenummer: 95112999.8
(22) Anmeldetag: 18.08.1995
(51) Int. Cl.: C09K 19/38, C09K 19/22, C09K 19/20, C07C 69/92, C07C 235/50, C07C 251/24, C08F 20/30, G02B 5/30, G02F 1/1335

(54) **Optisch aktive smektische photovernetzbare Flüssigkristalle**
Optically avtive smectic photo-curable liquid crystals
Cristaux liquides photo-réticulables smectiques optiquement actifs

(30) Priorität: 31.08.1994 CH 266694
(43) Veröffentlichungstag der Anmeldung: 06.03.1996
(73) Patentinhaber: Rolic AG, 6301 Zug (CH)
(72) Erfinder: Kelly, Stephen, CH-4313 Möhlin (CH); Schmitt, Klaus, D-79541 Lörrach (DE)
(74) Vertreter: England, Christopher David

(56) Entgegenhaltungen:
- EP-A- 0 611 981
- WO-A-93/22397
- DE-A- 4 233 660
- US-A- 5 202 053
- POLYM. INT. (PLYIEI,09598103);93; VOL.31 (1); PP.35-40, NAGOYA UNIV.;SCH. ENG.; NAGOYA; 464; JAPAN (JP), KAWAKAMI Y ET AL 'Synthesis and thermal transition of side-chain liquid-crystalline polyoxetanes having laterally attached mesogenic group'

## Beschreibung

Die vorliegende Erfindung betrifft optisch aktive, ferroelektrische, smektische photovernetzbare Flüssigkristalle, flüssigkristalline Gemische, die solche Verbindungen enthalten, sowie ihre Verwendung im vernetzten Zustand in optischen Bauelementen.

Photovernetzbare Flüssigkristalle versehen mit einer geeigneten Menge eines Photinitiators können, auf einem Substrat oder in einer Zelle, durch geeignete Orientierungsschichten oder in einem Feld orientiert werden und dann in diesem Zustand durch Bestrahlen mit Licht einer geeigneten Wellenlänge vernetzt werden. Die dadurch erzeugte Struktur bleibt auch bei hohen Temperaturen erhalten. Solche Schichten können beispielsweise Teile von Hybridschichten sein, wie sie in den Schweizerischen Patentanmeldungen CH 2016/94 und CH 2017/94 beschrieben sind. So lassen sich optische Bauelemente, wie zum Beispiel Wellenleiter, optische Gitter und Filter, Bauelemente mit piezoelektrischen und solche mit nicht-linearen optischen (NLO) Eigenschaften, usw. herstellen. Solche optische Bauelemente finden zum Beispiel in Frequenzverdoppelung (SHG) Einsatz.

Weitere Eigenschaften, wie beispielsweise die Doppelbrechung, die Brechungsindices, Transparenz, usw. müssen je nach Anwendungsgebiet unterschiedlichen Anforderungen genügen. Beispielsweise sollten Materalien für Frequenzverdoppelung (SHG) eine starke Koppelung zwischen der Richtung maximaler Hyperpolarisierbarkeit und der polaren Achse aufweisen, damit der Effizienzgrad der Signalverdoppelung hoch wird.

Die photovernetzbaren Flüssigkristalle müssen ausserdem eine gute chemische und thermische Stabilität, gute Löslichkeit in üblichen Lösungsmitteln und eine gute Stabilität gegenüber elektrischen Feldern und elektromagnetischer Strahlung besitzen. Weiterhin sollten sie von etwa 25°C bis etwa 100°C, insbesondere von etwa 25°C bis etwa 80°C eine geeignete Mesophase besitzen, beispielsweise sollten sie für die oben genannten Anwendungen eine breite chirale smektische Mesophase aufweisen.

Da Flüssigkristalle in der Regel als Mischungen mehrerer Komponenten zur Anwendung gelangen, ist es wichtig, dass die Komponenten untereinander gut mischbar sind. Komponenten, welche in Mischungen eine Frequenzverdoppelung bewirken oder zu optischer Aktivität führen, sind in der Regel selbst nicht flüssigkristallin. Sie haben daher in der Regel den Nachteil, dass sie zu einer starken Herabsetzung der Phasenübergangstemperaturen führen und daher nur in kleinen Konzentrationen eingesetzt werden können. Dies führt zu einem kleinen SHG-Effizienzgrad. Mischungen bestehend aus photovernetzbaren Flüssigkristallen und nicht photovernetzbaren Flüssigkristallen und/oder optisch aktiven (chiralen) Zusätzen erlauben eine Relaxierung der nicht vernetzten Bestandteile und führen dadurch zu einer Verminderung des SHG-Signals. Hingegen erlauben Netzwerke, in der die aktiven Komponenten eingebunden sind, hohe Konzentrationen dieser aktiven Zusätze. Gleichzeitig wird durch die Fixierung im Netzwerk die Orientierungsrelaxion verhindert. Um blaues oder grünes Licht erzeugen zu können, werden in solchen Netzwerken Farbstoffe eingesetzt, die bei der Wellenlänge des durch eine Laserdiode erzeugten SHG-Lichtes absorbieren. Solche Netzwerke haben den Nachteil, dass sie in der Regel keine ausreichende Langzeitstabilität aufweisen.

Er stellte sich somit die Aufgabe, photovernetzbare Verbindungen herzustellen, welche den obengenannten Anforderungen genügen, strukturierbar sind, eine ausgezeichnete thermische Stabilität und Langzeitstabilität aufweisen und einen hohen SHG-Effizienzgrad besitzen.

Die vorliegende Erfindung stellt nun Verbindungen, die in hervorragender Weise als Einzelkomponenten oder als Komponenten solcher Flüssigkristall-Mischungen geeignet sind, zur Verfügung. Es sind dies Verbindungen der allgemeinen Formel 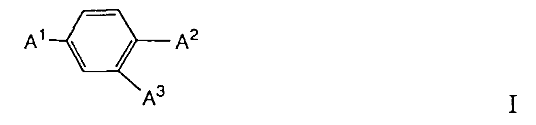 worin
- A¹, A²: je einen vernetzbaren mesogenen Rest, und
- A³: einen optisch aktiven, mesogenen Rest bedeuten.
Mesogene Reste sind dem Fachmann bekannt und beispielsweise in Flüssigkristalle in Tabellen, Deutscher Verlag für Grundstoffindustrie Leipzig, 1984, Band II" beschrieben. Damit die bekannten mesogenen Reste für obige Zwecke geeignet sind, werden sie endständig mit einem polymerisierbaren Rest versehen.

Da die erfindungsgemässen Verbindungen oder deren Mischungen eine getiltete smektische Phase aufweisen, können sie vor dem Vernetzen durch Anlegen eines elektrischen Feldes ausgerichtet werden. Dabei wird der Pitch abgewunden und eine einheitliche, ferroelektrische Schicht erzeugt.

Erfindungsgemäss sind die vernetzbaren, mesogenen Reste A¹ und A² gleich und Reste der Formel 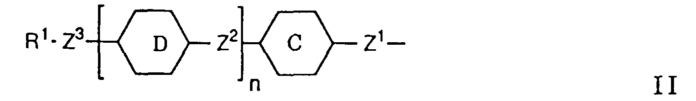 worin
- Ringe C und D: unabhängig voneinander gegebenenfalls mit Halogen, Methyl und/oder Cyano substituiertes 1,4-Phenylen, Pyridin-2,5-diyl, Pyrimidin-2,5-diyl, trans-1,4-Cyclohexylen oder trans-1,3-Dioxan-2,5-diyl;
- Z¹: eine Einfachbindung, -CH₂CH₂-, -CH₂O-, -COO-, -OOC-, -(CH₂)₄- oder -(CH₂)₃O- ;
- Z²: eine Einfachbindung, -CH₂CH₂-, -CH₂O-, -OCH₂-, -COO-, -OOC-, -(CH₂)₄-, -O(CH₂)₃- oder -(CH₂)₃O-;
- Z³: -(CH₂)ₘ-, -(CH₂)ₘO-, -(CH₂)ₘCOO-, oder -(CH₂)ₘOOC-;
- n: 0 oder 1;
- m: eine ganze Zahl von 1 bis 16; und
- R¹: eine vernetzbare Gruppe wie Acrylat, Methacrylat, 2-Chloracrylat, 2-Phenylacrylat, Acryloylphenylen, Acrylamid, Methacrylamid, 2-Phenylacrylamid, Epoxy, Itaconsäureester, Vinylether, Vinylester, Styrol-Derivate, Maleinsäure-Derivate, Fumarsäure-Derivate oder ein gegebenenfalls mit Methyl, Methoxy, Cyano und/oder Halogen substituiertes Zimtsäure-derivat bedeutend;
und es steht der optisch aktive, mesogene Rest A³ für einen Rest der allgemeinen Formel 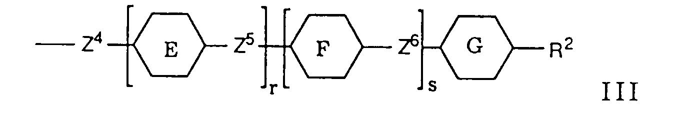 worin
- Ringe E und F: unabhängig voneinander gegebenenfalls mit Halogen, Methyl und/oder Cyano substituiertes 1,4-Phenylen, Pyridin-2,5-diyl, Pyrimidin-2,5-diyl, trans-1,4-Cyclohexylen oder trans-1,3-Dioxan-2,5-diyl;
- Ring G: unsubstituiertes oder mit Halogen, Methyl, Dimethylamino, Amino, Nitro und/oder Cyano mono- oder disubstituiertes 1,4-Phenylen;
- Z⁴: eine Einfachbindung, -(CH₂)ₚ-, -(CH₂)ₚO-, -O(CH₂)ₚ-, -(CH₂)ₚCOO-, -OOC(CH₂)ₚ-, -(CH₂)ₚOOC-, -COO(CH₂)ₚ-, -OOC(CH₂)ₚOOC-, -O(CH₂)ₚOOC- oder -COO(CH₂)ₚO- ;
- Z⁵, Z⁶: eine Einfachbindung, -CH₂CH₂-, -CH₂O-, -OCH₂-, -COO-, -OOC-, -(CH₂)₄-, -O(CH₂)₃- oder -(CH₂)₃O-;
- p: eine ganze Zahl von 1 bis 16;
- r, s: 0 oder 1; und
- R²: eine optisch aktive Gruppe wie (S)- bzw. (R)-2-Butyloxy, (S)- bzw. (R)-2-Pentyloxy, (S)- bzw. (R)-2-Hexyloxy, (S)- bzw. (R)-2-Heptyloxy, (S)- bzw. (R)-2-Octyloxy oder mit Methyl, Methoxy, Cyano und/oder Halogen substituiertes Alkyl, Alkoxy oder Alkanoyloxy mit 4 bis 8 Kohlenstoffatomen bedeuten.

Der Ausdruck "gegebenenfalls mit Halogen, Methyl und/oder Cyano substituiertes 1,4-Phenylen" bedeutet im Rahmen der vorliegenden Erfindung 1,4-Phenylen, 2-Fluor-1,4-phenylen, 3-Fluor-1,4-phenylen, 2,3-Difluor-1,4-phenylen, 2-Chlor-1,4-phenylen, 3-Chlor-1,4-phenylen, 2,3-2-Cyano-1,4-phenylen, 2-Cyano-1,4-phenylen, 3-Cyano-1,4-phenylen, 2,3-Dicyano-1,4-phenylen, 2-Methyl-1,4-phenylen, 3-Methyl-1,4-phenylen und dergleichen.

Der Ausdruck "unsubstituiertes oder mit Halogen, Methyl, Dimethylamino, Amino, Nitro und/oder Cyano mono- oder disubstituiertes 1,4-Phenylen" bedeutet im Rahmen der vorliegenden Erfindung 1,4-Phenylen oder beispielsweise 2-Amino-5-nitro-1,4-phenylen, 5-Amino-2-nitro-1,4-phenylen, 2-Dimethylamino-5-nitro-1,4-phenylen oder 5-Dimethylamino-2-nitro-1,4-phenylen und dergleichen.

Halogen steht für Fluor, Chlor, Brom und Jod, insbesondere für Fluor oder Chlor.

Der Ausdruck "mit Methyl, Methoxy, Cyano und/oder Halogen substituiertes Alkyl, Alkoxy oder Alkanoyloxy mit 4 bis 8 Kohlenstoffatomen" bedeutet im Rahmen der vorliegenden Erfindung optisch aktive Alkyl, Alkoxy oder Alkanoyloxygruppen wie beispielsweise (S)- bzw. (R)-2-Methylbutyl, (S)- bzw. (R)-2-Methylpentyl, (S)- bzw. (R)-2-Methylhexyl, (S)- bzw. (R)-2-Methylheptyl, (S)- bzw. (R)-2-Methoxybutyl, (S)- bzw. (R)-2-Methoxypentyl, (S)- bzw. (R)-2-Methoxyhexyl, (S)- bzw. (R)-2-Methoxyheptyl, (S)- bzw. (R)-1-Cyanobutyl, (S)- bzw. (R)-1-Cyanopentyl, (S)- bzw. (R)-1-Cyanohexyl, (S)- bzw. (R)-1-Cyanoheptyl, (S)- bzw. (R)-2-Cyanobutyl, (S)- bzw. (R)-2-Cyanopentyl, (S)- bzw. (R)-2-Cyanohexyl, (S)- bzw. (R)-2-Cyanoheptyl, (S)- bzw. (R)-1-Fluorbutyl, (S)- bzw. (R)-1-Fluorpentyl, (S)- bzw. (R)-1-Fluorhexyl, (S)- bzw. (R)-1-Fluorheptyl, (S)- bzw. (R)-2-Fluorbutyl, (S)- bzw. (R)-2-Fluorpentyl, (S)- bzw. (R)-2-Fluorhexyl, (S)- bzw. (R)-2-Fluorheptyl, (S)- bzw. (R)-1-Chlorbutyl, (S)- bzw. (R)-1-Chlorpentyl, (S)- bzw. (R)-1-Chlorhexyl, (S)- bzw. (R)-1-Chlorheptyl, (S)- bzw. (R)-2-Chlorbutyl, (S)- bzw. (R)-2-Chlorpentyl, (S)- bzw. (R)-2-Chlorhexyl oder (S)- bzw. (R)-2-Chlorheptyl und dergleichen.

Die erfindungsgemässen Verbindungen können auch durch die allgemeine Formel 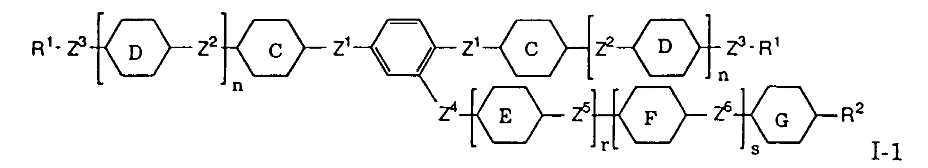 definiert werden, wobei die darin enthaltenen Symbole obengenannte Bedeutung haben.

Der Mesophasentyp der erfindungsgemässen Verbindungen der Formel I-1 kann durch Variation der Ringe C, D, E und F beeinflusst werden. So haben aromatische Ringe eher die Tendenz smektische Phasen zu erzeugen. Vorzugsweise bedeuten die Ringe C und D unabhängig voneinander 1,4-Phenylen, 2-Fluor-1,4-phenylen, 3-Fluor-1,4-phenylen, Pyridin-2,5-diyl oder Pyrimidin-2,5-diyl, insbesondere 1,4-Phenylen, 2-Fluor-1,4-phenylen oder 3-Fluor-1,4-phenylen.

Die Ringe E und F bedeuten in den erfindungsgemässen Verbindungen vorzugsweise 1,4-Phenylen, während der Ring G vorzugsweise 2-Amino-5-nitro-1,4-phenylen oder 5-Amino-2-nitro-1,4-phenylen bedeutet.

Besonders bevorzugte Verbindungen der Formel I-1 sind Verbindungen, worin Z¹ -CH₂O-, -COO- oder -OOC-; und Z² eine Einfachbindung, -CH₂CH₂-, -CH₂O-, -OCH₂-, -COO- oder -OOC- bedeuten.

In besonders bevorzugten Verbindungen der Formel I-1 bedeuten die Reste R¹ Acrylat, Methacrylat, 2-Chloracrylat, 2-Phenylacrylat, Acryloylphenylen, Acrylamid, Methacrylamid, 2-Phenylacrylamid, Epoxy, Vinylether, Vinylester, Styrol-Derivate, Maleinsäure-Derivate oder Fumarsäure-Derivate und dergleichen. Es sind dies somit Reste, welche nach Orientieren der Verbindungen der Formel I in einem Feld photochemisch vernetzt werden können.

Besonders bevorzugte Reste R¹ sind Acrylat, Methacrylat, Vinyloxy und Epoxy.

Als optisch aktive Reste R² werden besonders (S)- bzw. (R)-2-Butyloxy, (S)- bzw. (R)-2-Pentyloxy, (S)- bzw. (R)-2-Hexyloxy, (S)- bzw. (R)-2-Heptyloxy und (S)- bzw. (R)-2-Octyloxy verwendet.

Die mesogenen Reste A¹ und A² stehen vorzugsweise für einen Rest der Formel II, worin n = 0 ist, wie beispielsweise 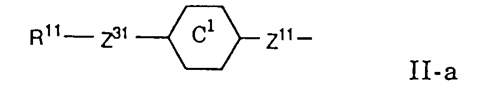 worin
- R¹¹: Acrylat, Methacrylat oder Epoxy;
- Z³¹: -(CH₂)ₜ-, -(CH₂)ₜO-, -(CH₂)ₜCOO- oder -(CH₂)ₜOOC-;
- t: eine ganze Zahl von 4 bis 12;
- Ring C¹: 1,4-Phenylen, 2-Fluor-1,4-phenylen, 3-Fluor-1,4-phenylen; und
- Z¹¹: -CH₂O-, -COO- oder -OOC- bedeuten.

Vorzugsweise bedeutet der mesogene Rest A³ einen Rest der Formel III, wie beispielsweise 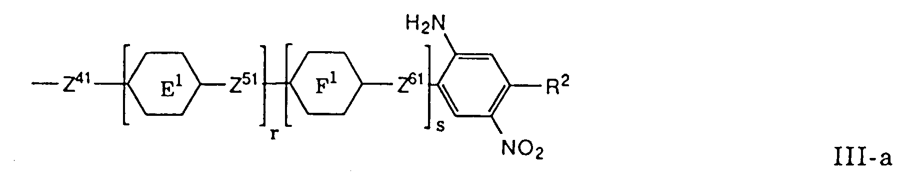 worin
- R²: (S)- bzw. (R)-2-Butyloxy, (S)- bzw. (R)-2-Pentyloxy, (S)- bzw. (R)-2-Hexyloxy, (S)- bzw. (R)-2-Heptyloxy und (S)- bzw. (R)-2-Octyloxy;
- r, s: 0 oder 1;
- Z⁴¹: -CH₂CH₂CH₂-, -OCH₂CH₂-, -CH₂CH₂O-, -COOCH₂- oder -CH₂OOC-;
- Z⁵¹, Z⁶¹: eine Einfachbindung, -CH₂CH₂-, -CH₂O-, -COO-, -OOC-, -(CH₂)₄- oder -(CH₂)₃O-; und
- Ringe E¹ und F¹: 1,4-Phenylen bedeuten.

Ganz besonders bevorzugte Verbindungen der Formel I-1 sind somit die Verbindungen der Formel 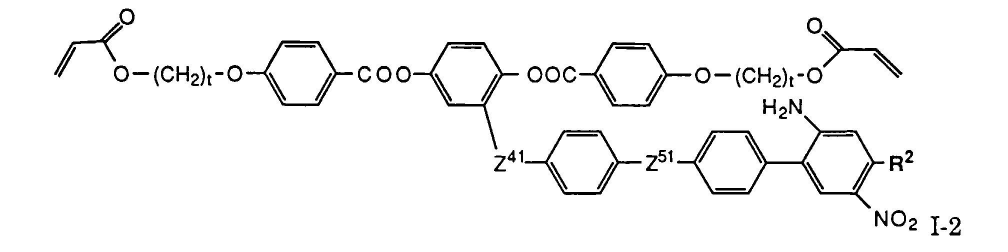 worin
- t: eine ganze Zahl von 4 bis 12 ist;
- R²: (S)- bzw. (R)-2-Butyloxy, (S)- bzw. (R)-2-Pentyloxy, (S)- bzw. (R)-2-Hexyloxy, (S)- bzw. (R)-2-Heptyloxy und (S)- bzw. (R)-2-Octyloxy;
- Z⁴¹: -COOCH₂-; und
- Z⁵¹: eine Einfachbindung, -COO- oder -OOC- bedeuten.

Die erfindungsgemässen Verbindungen der allgemeinen Formel I sind synthetisch sehr einfach zugänglich und können in an sich bekannter Weise, beispielsweise analog zu der in den Schemata 1 und 2 sowie in den Beispielen illustrierten Methode, hergestellt werden.

Die mesogenen Reste der Formel II und III sind bekannt oder Analoge bekannter Strukturen und können in an sich bekannter Weise hergestellt werden und anschliessend mit dem Aromaten verknüpft werden.

Eine kleine Menge BHT (2, 6-Di-tert.-butyl-4-methyl-phenol = "Butylhydroxytoluol") wird jeder Stufe beigemischt, um unerwünschtes thermisches Vernetzen zu unterbinden.

In den Schemata haben die Symbole die oben angegebenen Bedeutungen.

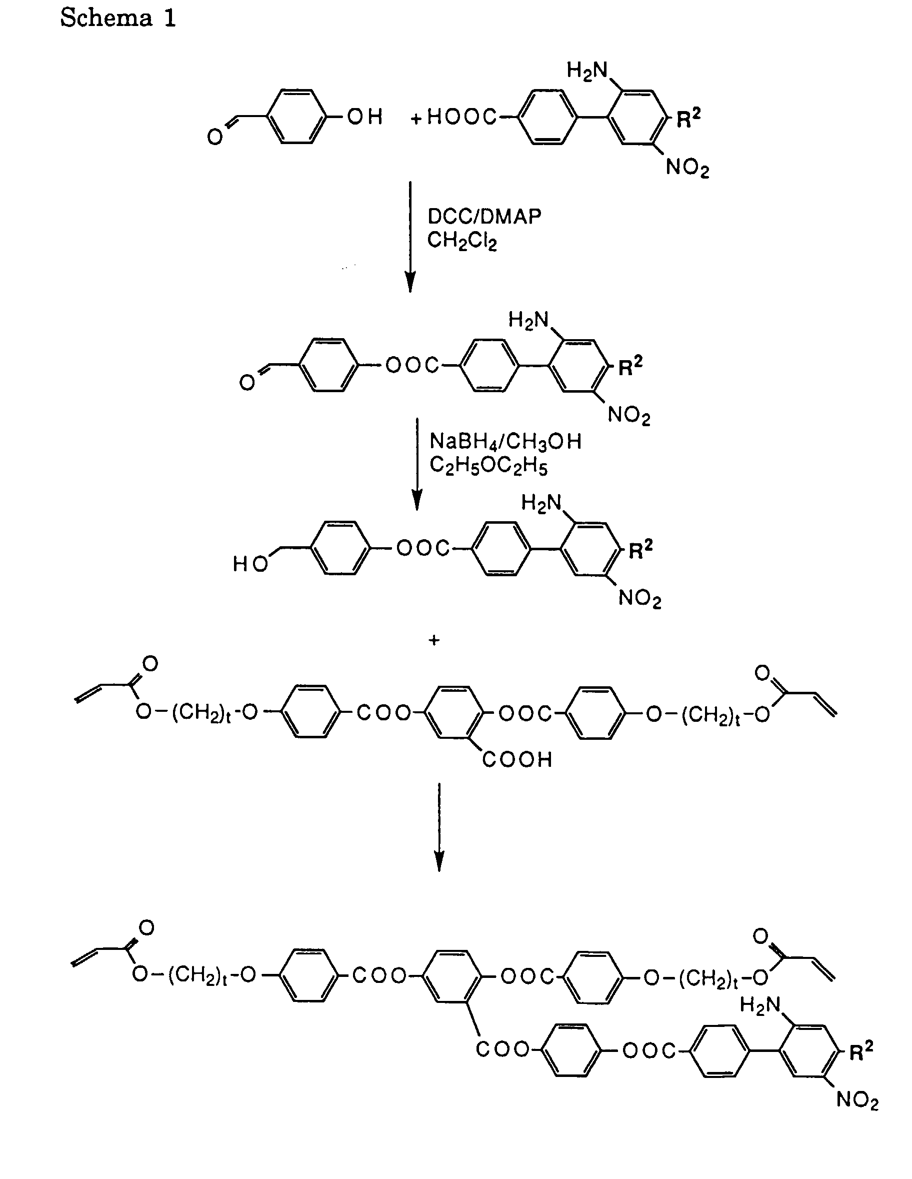

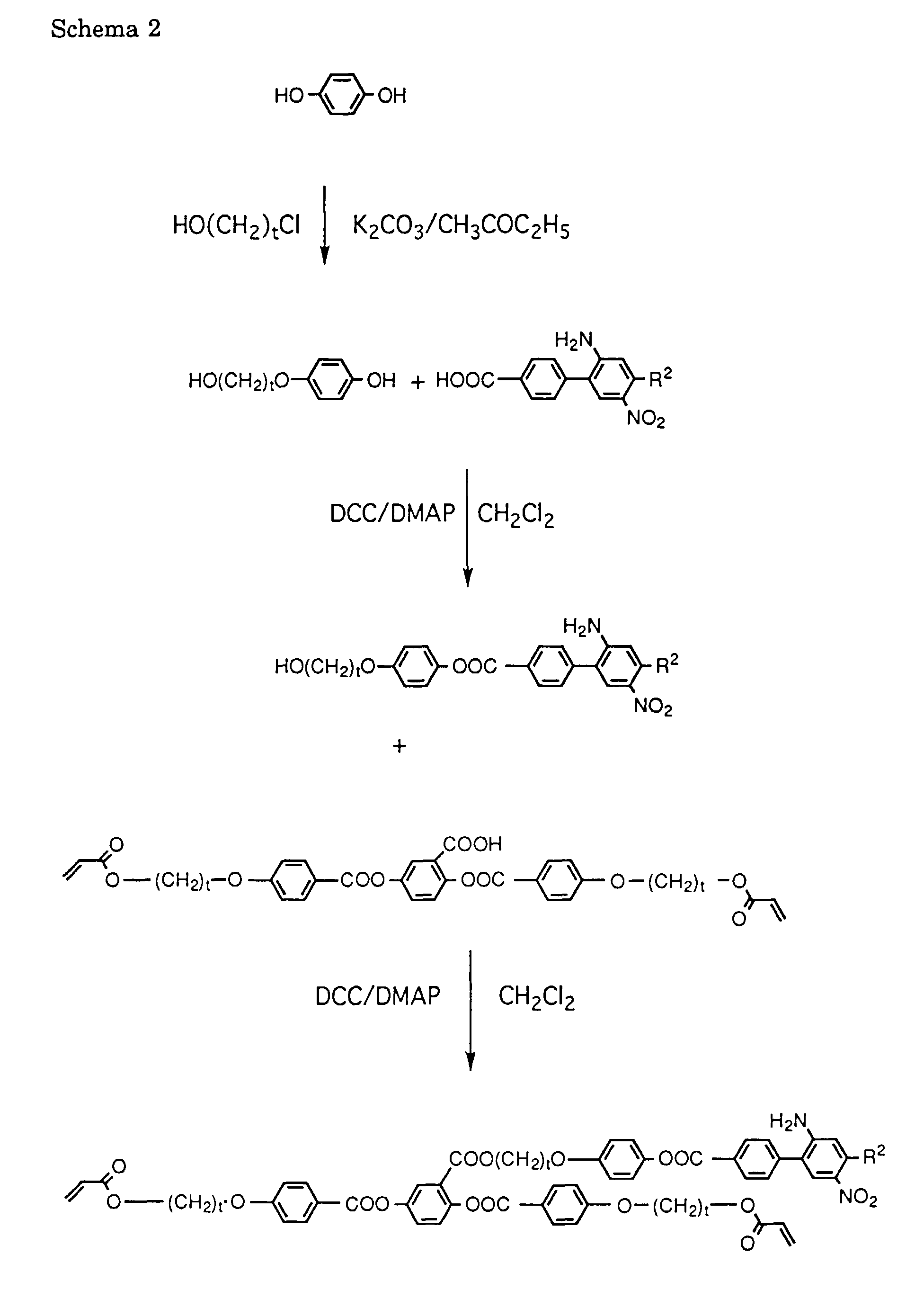

Die erfindungsgemässen Verbindungen der allgemeinen Formel I sind synthetisch einfach zugänglich und können beispielsweise in an sich bekannter Weise aus optisch aktiven Alkoholen (Derivaten der Formel III) und bis[2,5-bis(4-[n-Acryloyloxyalkyloxy)]phenylcarboxy)benzoesäuren hergestellt werden. Die Veresterung kann beispielsweise in Gegenwart von N,N'-Dicyclohexylcarbo-diimid (DCC) und 4-(Dimethylamino)pyridin (DMAP) in Dichlormethan oder einem anderen geeigneten Lösungsmittel wie z.B. Chloroform erfolgen.

Weiter können Verbindungen der Formel I hergestellt werden, indem zunächst 4-Benzaldehyd mit ω-Halo-1-alkohol-Derivaten veräthert, den Aldehyd mit Natriumborhydrid zum Benzylalkohol-Derivat der Formel II reduziert, dieses Derivat mit 2,5-Dihydoxybenzaldehyd in einer Mitsonobu-Reaktion veräthert, dann mit Acrylsäure verestert, mit Jones' Reagenz zur entsprechenden Benzoesäure oxidiert und anschliessend wie oben beschrieben mit optisch aktiven Alkoholen (Derivaten der Formel III) verestert wird.

Die optisch aktiven Alkohole (Derivate der Formel III) können beispielsweise aus dem Veresterungsprodukt von 4-Hydroxybenzaldehyd mit 4-[4-(2-Amino-5-nitro-4-[(S)-2-alkyloxy]phenyl)-benzoesäuren durch Reduktion mit Natriumborhydrid hergestellt werden.

Die Verbindungen der Formel I können als reine Verbindungen oder in Form von Gemischen untereinander und/oder mit anderen Flüssigkristallkomponenten verwendet werden.

Die erfindungsgemässen flüssigkristallinen Gemische enthalten mindestens 2 Komponenten, wovon mindestens eine Komponente eine Verbindung der Formel I ist. Eine zweite und gegebenenfalls weitere Komponenten können Verbindungen der Formel I oder auch andere, bekannte flüssigkristalline Verbindungen mit photovernetzbaren Gruppen sein. Es können auch weitere chirale Komponenten im Gemisch enthalten sein.

Aufgrund der guten Löslichkeit der Verbindungen der Formel I und aufgrund ihrer guten Mischbarkeit untereinander kann der Anteil an verschiedenen Verbindungen der Formel I in den erfindungsgemässen Gemischen hoch sein und bis 100 Gew.-% betragen.

Vorzugsweise enthalten die erfindungsgemässen Gemische neben einer oder mehreren Verbindungen der Formel I eine oder mehrere Verbindungen aus der Gruppe der Verbindungen der allgemeinen Formeln 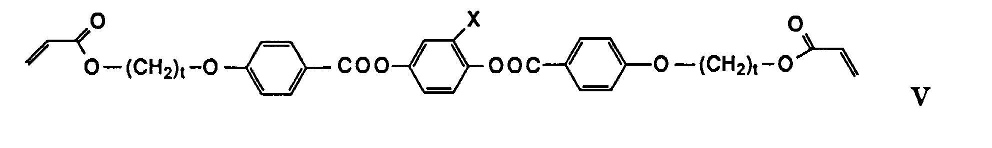 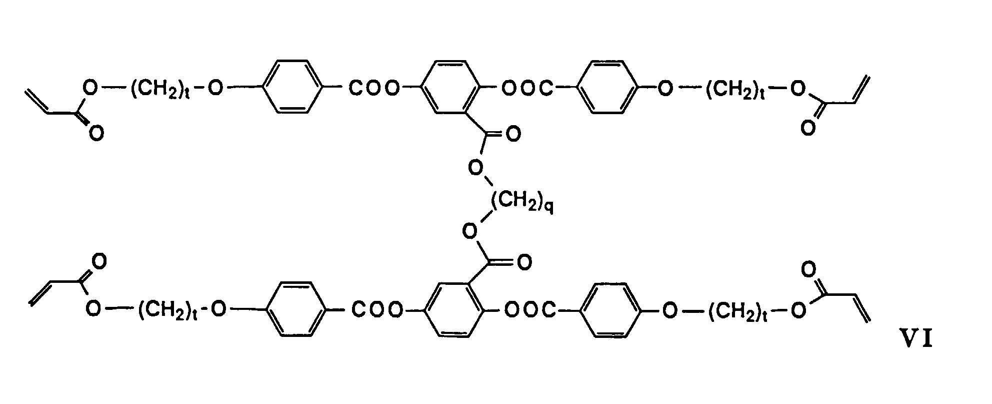 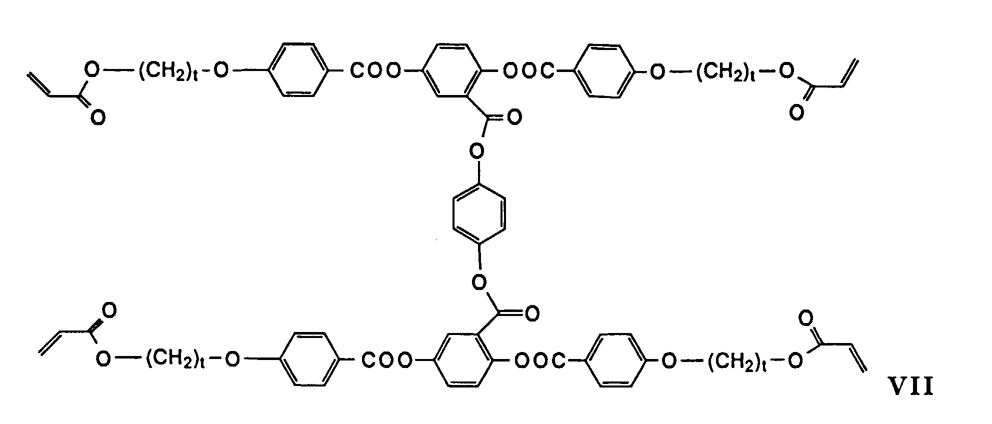 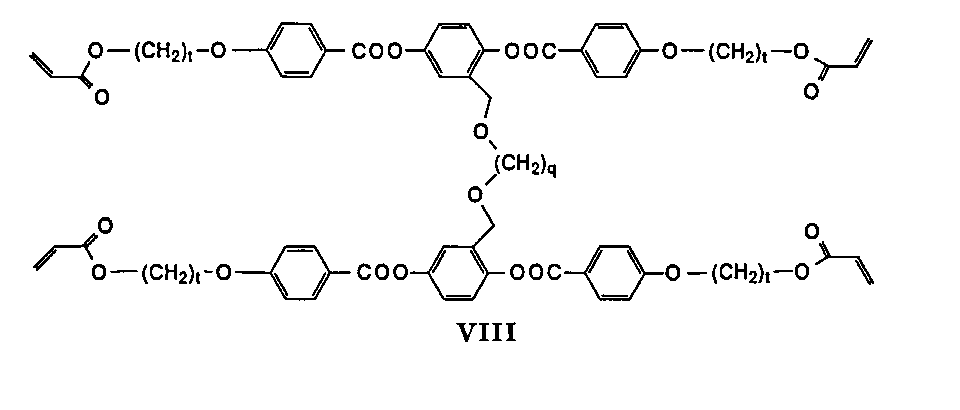 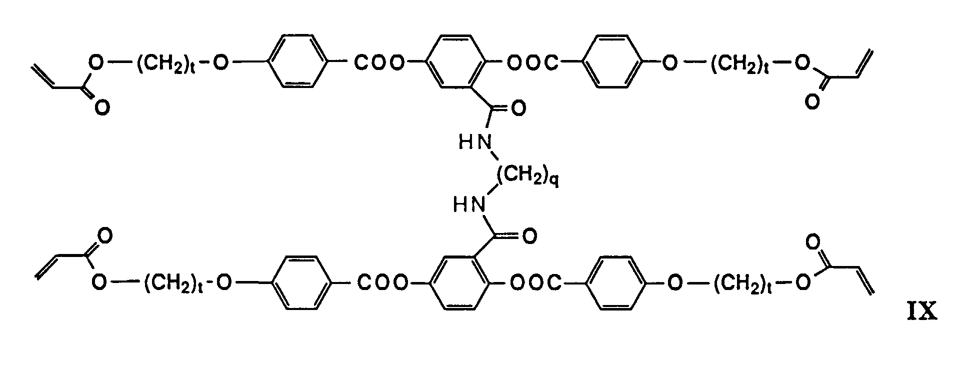 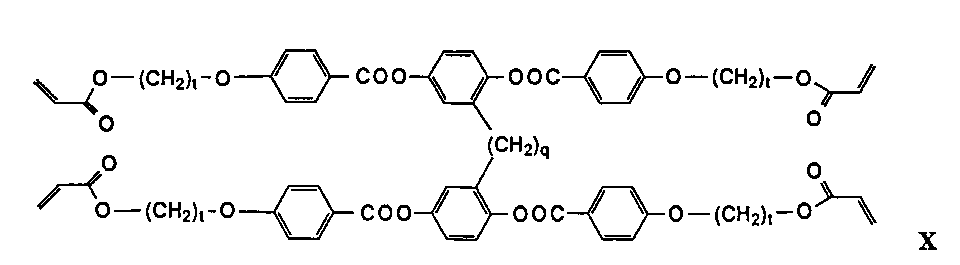 worin t eine ganze Zahl von 4 bis 12 und q eine ganze Zahl von 2 bis 12 bedeuten.

Die Herstellung der Verbindungen der Formel I sowie flüssigkristalliner Gemische enthaltend diese Verbindungen werden durch die folgenden Beispiele weiter veranschaulicht. C bedeutet eine kristalline, S eine smektische, N* die chiral nematische und I die isotrope Phase. "ac" bedeutet Wechselstrom und "dc" Gleichstrom. Optische Antipoden besitzen jeweils "spiegelbildliche Eigenschaften", d.h. gleiche Schmelzpunkte usw., führen aber zu entgegengesetztem Helixdrehsinn und entgegengesetzter Zirkularpolarisation des reflektierten Lichtes.

### Beispiel 1

Zu einer Lösung von 0,6 g 2,5-bis(4-[6-Acryloyloxyhexyloxy)]phenylcarboxy)benzoesäure, 0,4 g 4-[4-(2-Amino-5-nitro-4-[(S)-2-octyloxy]-phenyl)-benzoyloxy]phenylmethanol und 0,04 g 4-Dimethylaminopyridin in 20 ml Dichlormethan wurde unter Rühren bei Raumtemperatur 0,2 g N, N'-Dicyclodicyclohexyldicarbodiimid gegeben. Das Reaktionsgemisch wurde über Nacht bei Raumtemperatur gerührt, auf 100 ml Wasser gegossen und dann dreimal mit je 50 ml Dichlormethan extrahiert. Die organischen Phasen wurden zweimal mit je 100 ml Wasser gewaschen, über Magnesiumsulfat getrocknet, filtriert und anschliessend eingeengt. Chromatographische Reinigung des Rückstandes an Kieselgel mit Hexan/Ethylacetat (Vol. 1:1) und zweimaliges Umkristallisieren der gemäss Dünnschichtchromatographie reinen Fraktionen aus Aceton ergab 0,4 g (4-[4-(2-Amino-5-nitro-4-[(S)-2-octyloxy]phenyl)benzoyloxy]phenyl)-methyl 2,5-bis(4-[6-acryloyloxyhexyloxy)]phenylcarboxy)benzoesäureester; Smp. (C-I) 100°C, Sc*-N*, 96°C, Klp. (N*-I) 100°C.

Der als Ausgangsmaterial verwendete (4-[4-(5-Amino-2-nitro-4-[(S)-2-octyloxy]phenyl)benzoyloxy]phenyl)methanol wurde wie folgt hergestellt:
a) Ein Gemisch von 0,13 g Natriumborhydrid und 15 ml Wasser wurde tropfenweise bei 0°C mit einer Lösung von 0,8 g 4-[4-(2-Amino-5-nitro-4-[(S)-2-octyloxy]phenyl)benzoyloxy]benzaldehyd in 100 ml Dioxan versetzt. Das Reaktionsgemisch wurde weitere 60 Minuten bei 0°C und dann 10 Minuten bei Raumtemperatur gerührt und danach auf 100 ml Dichlormethan gegossen. Die organische Phase wurde abgetrennt und zweimal mit je 100 ml Wasser gewaschen. Die wässrigen Phasen wurden zweimal mit je 50 ml Dichlormethan extrahiert. Die vereinigten organischen Phasen wurden dann zweimal mit je 100 ml Wasser gewaschen, über Magnesiumsulfat getrocknet, die Suspension filtriert und das Filtrat eingeengt. Der Rückstand wurde ohne weitere Reinigung in die nächste Stufe eingesetzt.
b) Zu einer Lösung von 1,0 g 4-[4-(2-Amino-5-nitro-4-[(S)-2-octyloxy]-phenyl)benzoesäure, 0,3 g 4-Hydroxybenzaldehyd und 0,04 g 4-Dimethylaminopyridin in 20 ml Dichlormethan wurde unter Rühren bei Raumtemperatur 0,6 g N, N'-Dicyclodicyclohexyldicarbodiimid gegeben. Das Reaktionsgemisch wurde über Nacht bei Raumtemperatur gerührt, auf 100 ml Wasser gegossen und dann dreimal mit je 50 ml Dichlormethan extrahiert. Die organischen Phasen wurden zweimal mit je 100 ml Wasser gewaschen, über Magnesiumsulfat getrocknet, filtriert und das Filtrat anschliessend eingeengt. Chromatographische Reinigung des Rückstandes an Kieselgel mit Hexan/Ethylacetat (Vol. 1:1) und zweimaliges Umkristallisieren der gemäss Dünnschichtchromatographie reinen Fraktionen aus Ethanol ergab 0,9 g 4-[4-(2-Amino-5-nitro-4-[(S)-2-octyloxy]phenyl)benzoyloxy]benzaldehyd; Smp. (C-I) 77-78°C.

In analoger Weise können folgende Verbindungen hergestellt werden:
(4-[4-(2-Amino-5-nitro-4-[(S)-2-octyloxy]phenyl)benzoyloxy]phenyl)-methyl 2,5-bis(4-[7-acryloyloxyheptyloxy)]phenylcarboxy)benzoesäureester.
(4-[4-(2-Amino-5-nitro-4-[(S)-2-octyloxy]phenyl)benzoyloxy]phenyl)-methyl 2,5-bis(4-[8-acryloyloxyoctyloxy)]phenylcarboxy)benzoesäureester.
(4-[4-(2-Amino-5-nitro-4-[(S)-2-octyloxy]phenyl)benzoyloxy]phenyl)-methyl 2,5-bis(4-[9-acryloyloxynonyloxy)]phenylcarboxy)benzoesäureester.
(4-[4-(2-Amino-5-nitro-4-[(S)-2-octyloxy]phenyl)benzoyloxy]phenyl)-methyl 2,5-bis(4-[10-acryloyloxydecyloxy)]phenylcarboxy)benzoesäureester.
(4-[4-(2-Amino-5-nitro-4-[(S)-2-octyloxy]phenyl)benzoyloxy]phenyl)-methyl 2,5-bis(4-[11-acryloyloxyundecyloxy)]phenylcarboxy)benzoesäureester; Smp. (C-SA) 108°C; Klp. (SA-I) 112°C.
(4-[4-(2-Amino-5-nitro-4-[(S)-2-octyloxy]phenyl)benzoyloxy]phenyl)-methyl 2,5-bis(4-[12-acryloyloxydodecyloxy)]phenylcarboxy)benzoesäureester.
[4-(2-Amino-5-nitro-4-[(S)-2-octyloxy]benzoyloxy)phenyl]methyl 2,5-bis(4-[7-acryloyloxyheptyloxy)]phenylcarboxy)benzoesäureester.
[4-(2-Amino-5-nitro-4-[(S)-2-octyloxy]benzoyloxy)phenyl]methyl 2,5-bis(4-[8-acryloyloxyoctyloxy)]phenylcarboxy)benzoesäureester.
[4-(2-Amino-5-nitro-4-[(S)-2-octyloxy]benzoyloxy)phenyl]methyl 2,5-bis(4-[9-acryloyloxynonyloxy)]phenylcarboxy)benzoesäureester.
[4-(2-Amino-5-nitro-4-[(S)-2-octyloxy]benzoyloxy)phenyl]methyl 2,5-bis(4-[10-acryloyloxydecyloxy)]phenylcarboxy)benzoesäureester.
[4-(2-Amino-5-nitro-4-[(S)-2-octyloxy]benzoyloxy)phenyl]methyl 2,5-bis(4-[11-acryloyloxyundecyloxy)]phenylcarboxy)benzoesäureester.
[4-(2-Amino-5-nitro-4-[(S)-2-octyloxy]benzoyloxy)phenyl]methyl 2,5-bis(4-[12-acryloyloxydodecyloxy)]phenylcarboxy)benzoesäureester.

### Beispiel 2

0,6 g 2,5-bis(4-[6-Acryloyloxyhexyloxy)]phenylcarboxy)benzoesäure und 0,4 g (4-[4-(2-Amino-5-nitro-4-[(S)-2-octyloxy]phenyl)benzoyloxy]phenoxy)-ethanol, 0,04 g 4-Dimethylaminopyridin, 0,2 g N, N'-Dicyclodicyclohexyldicarbodiimid und 20 ml Dichlormethan werden in analoger Weise zu Beispiel 1 zu (4-[4-(2-Amino-5-nitro-4-[(S)-2-octyloxy]phenyl)benzoyloxy]-phenoxy)ethyl 2,5-bis(4-[6-acryloyloxyhexyloxy)]phenylcarboxy)benzoesäureester umgesetzt.

Der als Ausgangsmaterial verwendete (4-[4-(2-Amino-5-nitro-4-[(S)-2-octyloxy]phenyl)benzoyloxy]phenoxy)ethanol wird wie folgt hergestellt:
a) 0,6 g 4-[4-(2-Amino-5-nitro-4-[(S)-2-octyloxy]phenyl)benzoesäure und 0,2 g (4-Hydroxyphenoxy)ethanol, 0,04 g 4-DimethylAminopyridin, 0,4 g N, N'-Dicyclodicyclohexyldicarbodiimid und 50 ml Dichlormethan werden in analoger Weise zu Beispiel 1 zu 0,4 g (4-[4-(2-Amino-5-nitro-4-[(S)-2-octyloxy]phenyl)benzoyloxy]phenoxy)ethanol umgesetzt.
b) Eine Lösung von 5,0 g 4-Hydrochinon und 3,7 g Chlorethanol in 150 ml Ethyl-methylketon wird mit 25 g fein pulverisiertem Kaliumcarbonat versetzt und das Gemisch über Nacht unter leichtem Rückfluss erwärmt. Die Suspension wird genutscht und das Filtrat im Vakuum eingeengt. Eine Lösung des Rückstands in 100 ml Diethylether wird zweimal mit je 100 ml Wasser gewaschen, über Natriumsulfat getrocknet und eingeengt. Chromatographie des Rohproduktes an Kieselgel mit Hexan/Ethylacetat (Vol. 7:3) ergibt 2,0 g (4-Hydroxyphenoxy)ethanol.

In analoger Weise können folgende Verbindungen hergestellt werden:
(4-[4-(2-Amino-5-nitro-4-[(S)-2-octyloxy]phenyl)benzoyloxy]phenoxy)ethyl 2,5-bis(4-[7-acryloyloxyheptyloxy)]phenylcarboxy)benzoesäureester.
(4-[4-(2-Amino-5-nitro-4-[(S)-2-octyloxy]phenyl)benzoyloxy]phenoxy)ethyl 2,5-bis(4-[8-acryloyloxyoctyloxy)]phenylcarboxy)benzoesäureester.
(4-[4-(2-Amino-5-nitro-4-[(S)-2-octyloxy]phenyl)benzoyloxy]phenoxy)ethyl 2,5-bis(4-[9-acryloyloxynonyloxy)]phenylcarboxy)benzoesäureester.
(4-[4-(2-Amino-5-nitro-4-[(S)-2-octyloxy]phenyl)benzoyloxy]phenoxy)ethyl 2,5-bis(4-[10-acryloyloxydecyloxy)]phenylcarboxy)benzoesäureester.
(4-[4-(2-Amino-5-nitro-4-[(S)-2-octyloxy]phenyl)benzoyloxy]phenoxy)ethyl 2,5-bis(4-[11-acryloyloxyundecyloxy)]phenylcarboxy)benzoesäureester.
(4-[4-(2-Amino-5-nitro-4-[(S)-2-octyloxy]phenyl)benzoyloxy]phenoxy)ethyl 2,5-bis(4-[12-acryloyloxydodecyloxy)]phenylcarboxy)benzoesäureester.
[4-(2-Amino-5-nitro-4-[(S)-2-octyloxy]benzoyloxy)phenoxy]ethyl 2,5-bis(4-[7-acryloyloxyheptyloxy)]phenylcarboxy)benzoesäureester.
[4-(2-Amino-5-nitro-4-[(S)-2-octyloxy]benzoyloxy)phenoxy]ethyl 2,5-bis(4-[8-acryloyloxyoctyloxy)]phenylcarboxy)benzoesäureester.
[4-(2-Amino-5-nitro-4-[(S)-2-octyloxy]benzoyloxy)phenoxy]ethyl 2,5-bis(4-[9-acryloyloxynonyloxy)]phenylcarboxy)benzoesäureester.
[4-(2-Amino-5-nitro-4-[(S)-2-octyloxy]benzoyloxy)phenoxy]ethyl 2,5-bis(4-[10-acryloyloxydecyloxy)]phenylcarboxy)benzoesäureester.
[4-(2-Amino-5-nitro-4-[(S)-2-octyloxy]benzoyloxy)phenoxy]ethyl 2,5-bis(4-[11-acryloyloxyundecyloxy)]phenylcarboxy)benzoesäureester.
[4-(2-Amino-5-nitro-4-[(S)-2-octyloxy]benzoyloxy)phenoxy]ethyl 2,5-bis(4-[12-acryloyloxydodecyloxy)]phenylcarboxy)benzoesäureester.

### Beispiel 3

0,3 g 4-[4-(2-Amino-5-nitro-4-[(S)-2-octyloxy]phenyl)benzoesäure, 0,8 g 4-Hydroxybutyl 2,5-bis(4-[11-acryloyloxyundecyloxy)]phenylcarboxy)benzoesäureester und 0,04 g 4-Dimethylaminopyridin, 0,2 g N, N'-Dicyclodicyclohexyldicarbodiimid und 50 ml Dichlormethan werden in analoger Weise zu Beispiel 1 zu 0,5 g 4-[4-(2-Amino-5-nitro-4-[(S)-2-octyloxy]phenyl)-benzoyloxy]butyl 2,5-bis(4-[11-acryloyloxyundecyloxy)]phenylcarboxy)-benzoesäureester umgesetzt.

Der als Ausgangsmaterial verwendete 4-Hydroxybutyl 2,5-bis(4-[11-acryloyloxyundecyloxy)]phenylcarboxy)benzoesäureester wird wie folgt hergestellt:
(a) 1,0 g 2,5-bis(4-[11-Acryloyloxyundecyloxy)]phenylcarboxy)benzoesäure und 1,0 g 1, 4-Butandiol, 0,04 g 4-Dimethylaminopyridin, 0,4 g N, N'-Dicyclodicyclohexyldicarbodiimid und 50 ml Dichlormethan werden in analoger Weise zu Beispiel 1 zu 0,8 g 4-Hydroxybutyl 2,5-bis(4-[11-acryloyloxyundecyloxy)]phenylcarboxy)benzoesäureester umgesetzt.

In analoger Weise können folgende Verbindungen hergestellt werden:
3-[4-(2-Amino-5-nitro-4-[(S)-2-octyloxy]phenyl)benzoyloxy]propyl 2,5-bis(4-[11-acryloyloxyundecyloxy)]phenylcarboxy)benzoesäureester;
5-[4-(2-Amino-5-nitro-4-[(S)-2-octyloxy]phenyl)benzoyloxy]pentyl 2,5-bis(4-[11-acryloyloxyundecyloxy)]phenylcarboxy)benzoesäureester;
6-[4-(2-Amino-5-nitro-4-[(S)-2-octyloxy]phenyl)benzoyloxy]hexyl 2,5-bis(4-[11-acryloyloxyundecyloxy)]phenylcarboxy)benzoesäureester;
7-[4-(2-Amino-5-nitro-4-[(S)-2-octyloxy]phenyl)benzoyloxy]heptyl 2,5-bis(4-[11-acryloyloxyundecyloxy)]phenylcarboxy)benzoesäureester;
8-[4-(2-Amino-5-nitro-4-[(S)-2-octyloxy]phenyl)benzoyloxy]octyl 2,5-bis(4-[11-acryloyloxyundecyloxy)]phenylcarboxy)benzoesäureester;
9-[4-(2-Amino-5-nitro-4-[(S)-2-octyloxy]phenyl)benzoyloxy]nonyl 2,5-bis(4-[11-acryloyloxyundecyloxy)]phenylcarboxy)benzoesäureester;
10-[4-(2-Amino-5-nitro-4-[(S)-2-octyloxy]phenyl)benzoyloxy]decyl 2,5-bis(4-[11-acryloyloxyundecyloxy)]phenylcarboxy)benzoesäureester;
11-[4-(2-Amino-5-nitro-4-[(S)-2-octyloxy]phenyl)benzoyloxy]undecyl 2,5-bis(4-[11-acryloyloxyundecyloxy)]phenylcarboxy)benzoesäureester;
12-[4-(2-Amino-5-nitro-4-[(S)-2-octyloxy]phenyl)benzoyloxy]dodecyl 2,5-bis(4-[11-acryloyloxyundecyloxy)]phenylcarboxy)benzoesäureester;
3-[4-(2-Amino-5-nitro-4-[(S)-2-octyloxy]phenyl)benzoyloxy]propyl 2,5-bis(4-[5-acryloyloxypentyloxy)]phenylcarboxy)benzoesäureester;
3-[4-(2-Amino-5-nitro-4-[(S)-2-octyloxy]phenyl)benzoyloxy]propyl 2,5-bis(4-[6-acryloyloxyhexyloxy)]phenylcarboxy)benzoesäureester;
3-[4-(2-Amino-5-nitro-4-[(S)-2-octyloxy]phenyl)benzoyloxy]propyl 2,5-bis(4-[7-acryloyloxyheptyloxy)]phenylcarboxy)benzoesäureester;
3-[4-(2-Amino-5-nitro-4-[(S)-2-octyloxy]phenyl)benzoyloxy]propyl 2,5-bis(4-[8-acryloyloxyoctyloxy)]phenylcarboxy)benzoesäureester;
3-[4-(2-Amino-5-nitro-4-[(S)-2-octyloxy]phenyl)benzoyloxy]propyl 2,5-bis(4-[9-acryloyloxynonyloxy)]phenylcarboxy)benzoesäureester;
3-[4-(2-Amino-5-nitro-4-[(S)-2-octyloxy]phenyl)benzoyloxy]propyl 2,5-bis(4-[10-acryloyloxydecyloxy)]phenylcarboxy)benzoesäureester;

### Beispiel 4

Ein Gemisch (I-N*, 114°C, N*-Sc* 106°C und Sc*-Sx 70°C) aus 0,05 g 4-[4-(5-Amino-2-nitro-4-[(S)-2-octyloxy]phenyl)benzoyloxy]phenylmethyl 2,5-bis(4-[6-acryloyloxyhexyloxy)]phenylcarboxy)benzoesäureester und 0,20 g 2,5-bis(4-[6-Acryloyloxyhexyloxy)]phenylcarboxy)benzol versehen mit 6 µm Spacer wurde vorgelegt, mit 3 Gew.% eines Photoinitiators (Irgacure, Ciba Geigy) und 1 Gew.% BHT versetzt und bei 160°C in eine 7.8 µm Zelle mit paralleler Orientierung eingefüllt. Die Schicht wurde bei 105°C in einem elektrischen Feld (15 V ac) orientiert, dann bei 90°C und bei 110 V dc die Helix abgewunden und in diesem Zustand mit Xenon-Licht beleuchtet (zB. 15 Minuten). Diese Schicht fungiert als ein Frequenzverdoppler.

## Patentansprüche

1. Verbindungen der allgemeinen Formel 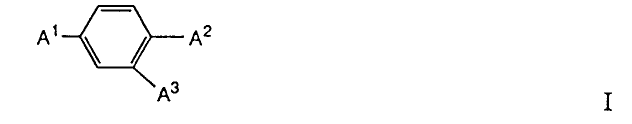 worin
A¹, A² für je einen vernetzbaren, mesogenen Rest, und stehen, wobei
A³ für einen optisch aktiven, mesogenen Rest stehen, wobei die mesogenen Reste A¹ und A² gleich sind und Reste der Formel 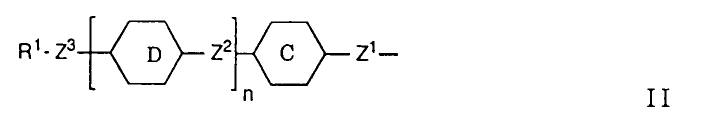
bedeuten, worin
Ringe C und D unabhängig voneinander gegebenenfalls mit Halogen, Methyl und/oder Cyano substituiertes 1,4-Phenylen, Pyridin-2,5-diyl, Pyrimidin-2,5-diyl, trans-1,4-Cyclohexylen oder trans-1,3-Dioxan-2,5-diyl;
Z¹ eine Einfachbindung, -CH₂CH₂-, -CH₂O-, -COO-, -OOC-, -(CH₂)₄- oder -(CH₂)₃O-;
Z² eine Einfachbindung, -CH₂CH₂-, -CH₂O-, -OCH₂-, -COO-, -OOC-, -(CH₂)₄-, -O(CH₂)₃- oder -(CH₂)₃O-;
Z³ -(CH₂)ₘ-, -(CH₂)ₘO-, -(CH₂)ₘCOO- oder -(CH₂)ₘOOC-;
n 0 oder 1;
m eine ganze Zahl von 1 bis 16; und
R¹ eine vernetzbare Gruppe wie Acrylat, Methacrylat, 2-Chloracrylat, 2-Phenylacrylat, Acryloylphenylen, Acrylamid, Methacrylamid, 2-Phenylacrylamid, Epoxy, Itaconsäureester, Vinylether, Vinylester, Styrol-Derivate, Maleinsäure-Derivate, Fumarsäure-Derivate oder ein gegebenenfalls mit Methyl, Methoxy, Cyano und/oder Halogen substituiertes Zimtsäurederivat bedeuten; und wobei der mesogene Rest A³ für einen Rest der Formel 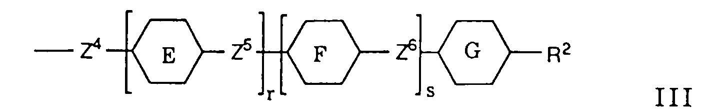
steht, worin
Ringe E und F unabhängig voneinander gegebenenfalls mit Halogen, Methyl und/oder Cyano substituiertes 1,4-Phenylen, Pyridin-2,5-diyl, Pyrimidin-2,5-diyl, trans-1,4-Cyclohexylen oder trans-1,3-Dioxan-2,5-diyl;
Ring G unsubstituiertes oder mit Halogen, Methyl, Dimethylamino, Amino, Nitro und/oder Cyano mono- oder disubstituiertes 1,4-Phenylen;
Z⁴ eine Einfachbindung, -(CH₂)ₚ-, -(CH₂)ₚO-, -O(CH₂)ₚ-, -(CH₂)ₚCOO-, -OOC(CH₂)ₚ-, -(CH₂)ₚOOC-, -COO(CH₂)ₚ-, -OOC(CH₂)ₚOOC-, -O(CH₂)ₚOOC- oder -COO(CH₂)ₚO- ;
Z⁵, Z⁶ eine Einfachbindung, -CH₂CH₂-, -CH₂O-, -OCH₂-, -COO-, -OOC-, -(CH₂)₄-, -O(CH₂)₃- oder -(CH₂)₃O- ;
p eine ganze Zahl von 1 bis 16;
r, s 0 oder 1; und
R² eine optisch aktive Gruppe wie (S)- bzw. (R)-2-Butyloxy, (S)- bzw. (R)-2-Pentyloxy, (S)- bzw. (R)-2-Hexyloxy, (S)-bzw. (R)-2-Heptyloxy, (S)- bzw. (R)-2-Octyloxy oder mit Methyl, Methoxy, Cyano und/oder Halogen substituiertes Alkyl, Alkoxy oder Alkanoyloxy mit 4 bis 8 Kohlenstoffatomen bedeuten.

2. Verbindungen gemäss Anspruch 1, dadurch gekennzeichnet, dass die mesogenen Reste A¹ und A² einen Rest der Formel 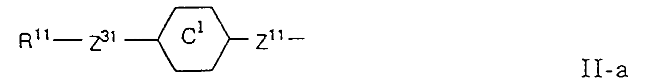 bedeuten, worin
R¹¹ Acrylat, Methacrylat oder Epoxy;
Z³¹ -(CH₂)ₜ-, -(CH₂)ₜO-, -(CH₂)ₜCOO- oder -(CH₂)ₜOOC- ;
t eine ganze Zahl von 4 bis 12;
Ring C¹ 1,4-Phenylen, 2-Fluor-1,4-phenylen, 3-Fluor-1,4-phenylen; und
Z¹¹ -CH₂O-, -COO- oder -OOC- bedeuten.

3. Verbindungen gemäss Anspruch 1, dadurch gekennzeichnet, dass der optisch aktive, mesogene Rest A³ einen Rest der Formel 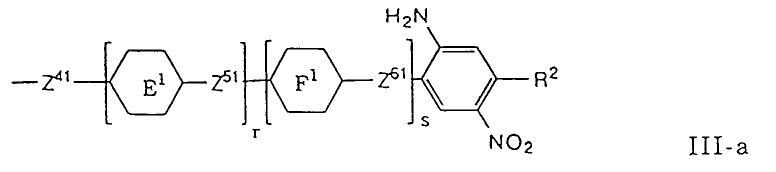 bedeutet, worin
R² (S)- bzw. (R)-2-Butyloxy, (S)- bzw. (R)-2-Pentyloxy, (S)-bzw. (R)-2-Hexyloxy, (S)- bzw. (R)-2-Heptyloxy oder (S)- bzw. (R)-2-Octyloxy;
r, s 0 oder 1;
Z⁴¹ -CH₂CH₂CH₂-, -OCH₂CH₂-, -CH₂CH₂O-, -COOCH₂- oder -CH₂OOC-;
Z⁵¹, Z⁶¹ eine Einfachbindung, -CH₂CH₂-, -CH₂O-, -COO-, -OOC-, -(CH₂)₄- oder -(CH₂)₃O-; und
Ringe E¹ und F¹ 1,4-Phenylen bedeuten.

4. Verbindungen der Formel 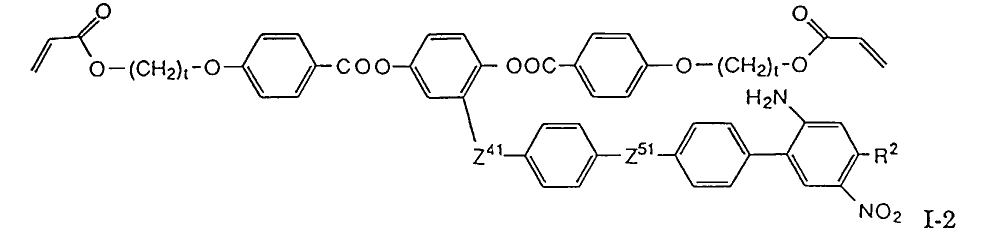 worin
t eine ganze Zahl von 4 bis 12 ist;
R² (S)- bzw. (R)-2-Butyloxy, (S)- bzw. (R)-2-Pentyloxy, (S)- bzw. (R)-2-Hexyloxy, (S)- bzw. (R)-2-Heptyloxy oder (S)- bzw. (R)-2-Octyloxy;
Z⁴¹ -COOCH₂-; und
Z⁵¹ eine Einfachbindung, -COO- oder -OOC- bedeuten.

5. Vernetzbare, flüssigkristalline Gemische bestehend aus mindestens 2 Komponenten, wovon mindestens eine Komponente eine Verbindung der in Anspruch 1 definierten Formel I ist.

6. Vernetzbare, flüssigkristalline Gemische gemäss Anspruch 5, dadurch gekennzeichnet, dass sie neben einer oder mehreren Verbindungen der Formel I, eine oder mehrere Verbindungen aus der Gruppe der Verbindungen der Formeln 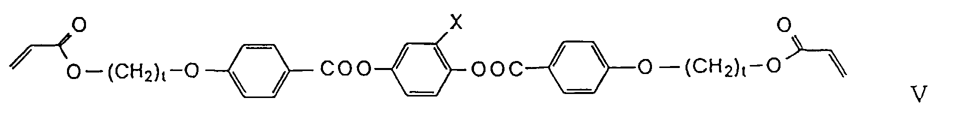 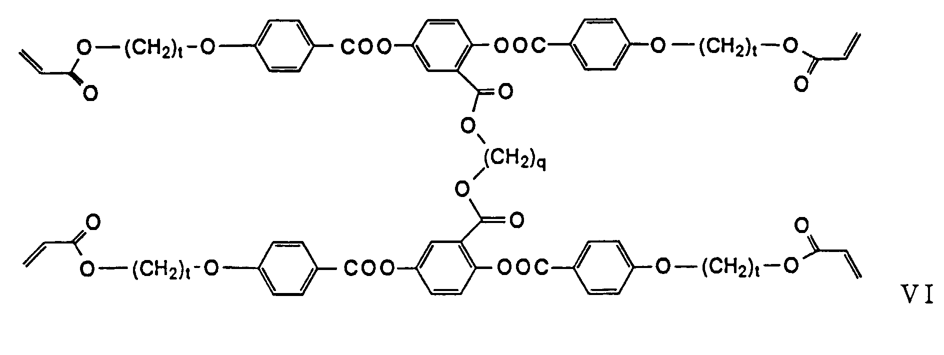 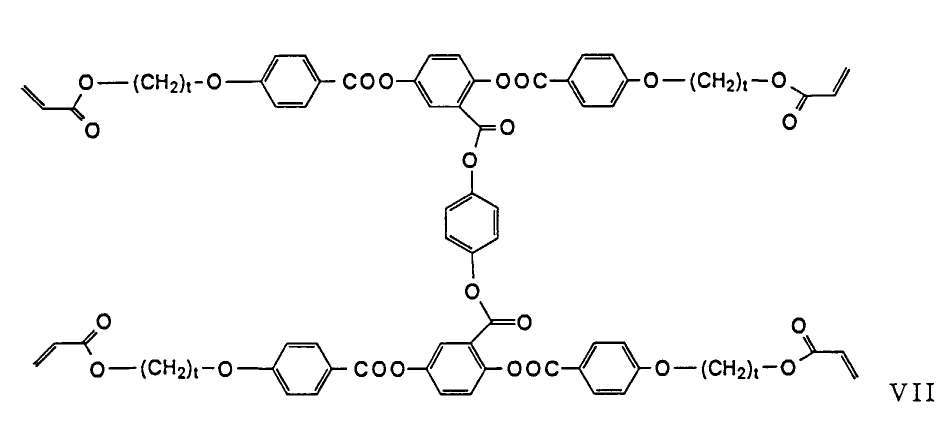 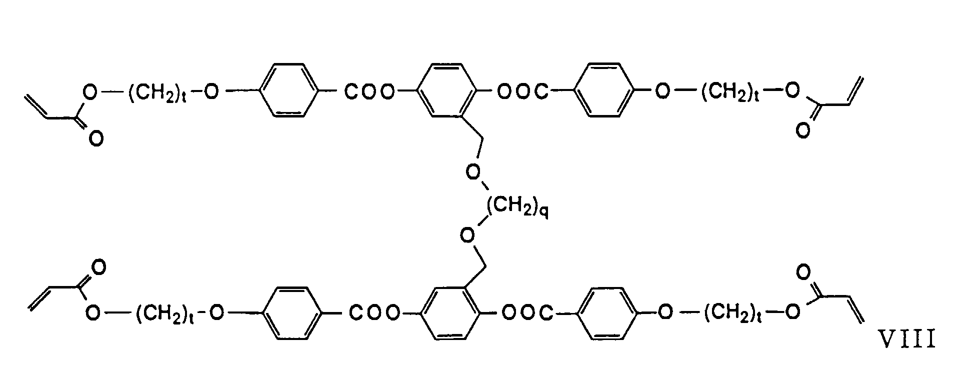 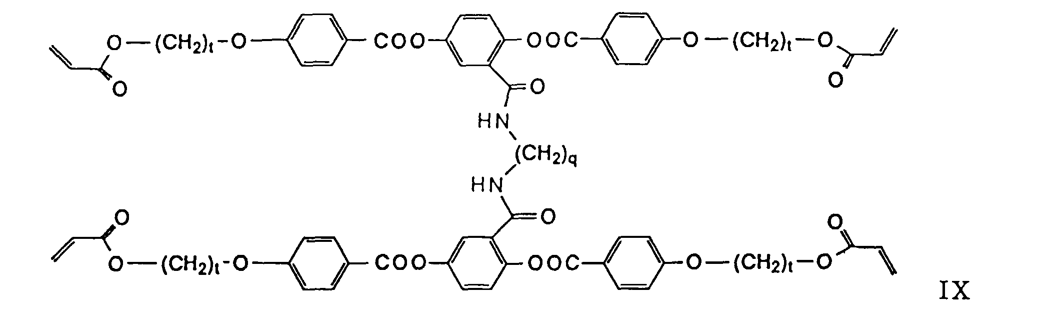 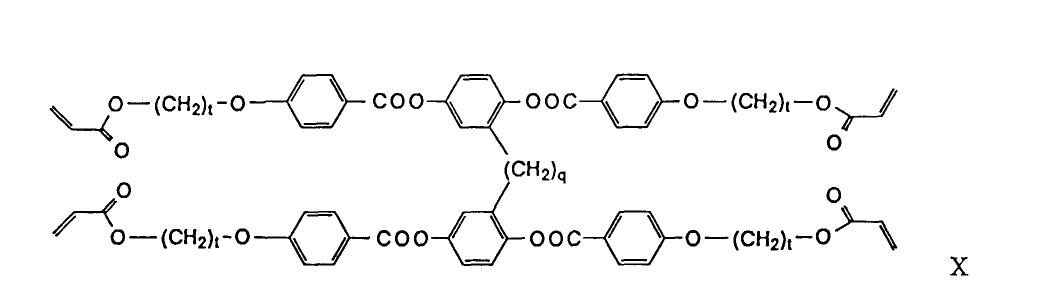 worin t eine ganze Zahl von 4 bis 12 und q eine ganze Zahl von 2 bis 12 bedeuten.

7. Verwendung von Verbindungen gemäss einem der Ansprüche 1 bis 4 in ihrem vernetzten Zustand für optische Bauelemente.

8. Verwendung von vernetzbaren flüssigkristallinen Gemischen gemäss Anspruch 5 oder 6 in ihrem vernetzten Zustand für optische Bauelemente.

## Claims

1. Compounds of the general formula 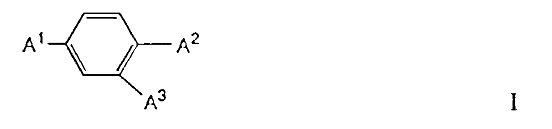 wherein
A¹ and A² each represents a crosslinkable mesogenic radical, and
A³ represents an optically active, mesogenic radical,
wherein the mesogenic radicals A¹ and A² are the same and represent radicals of the formula 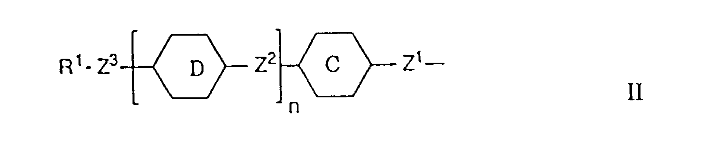 wherein
rings C and D each independently of the other represents 1,4-phenylene optionally substituted by halogen, methyl and/or by cyano, pyridine-2,5-diyl, pyrimidine-2,5-diyl, trans-1,4-cyclohexylene or trans-1,3-dioxane-2,5-diyl;
Z¹ represents a single bond, -CH₂CH₂-, -CH₂O-, -COO-, -OOC-, -(CH₂)₄- or -(CH₂)₃O-;
Z² represents a single bond, -CH₂CH₂-, -CH₂O-, -OCH₂-, -COO-, -OOC-, -(CH₂)₄-, -O(CH₂)₃- or -(CH₂)₃O-;
Z³ represents -(CH₂)ₘ-, -(CH₂)ₘO-, -(CH₂)ₘCOO- or -(CH₂)ₘOOC-;
n is 0 or 1;
m is an integer from 1 to 16; and
R¹ represents a crosslinkable group such as acrylate, methacrylate, 2-chloroacrylate, 2-phenylacrylate, acryloylphenylene, acrylamide, methacrylamide, 2-phenylacrylamide, epoxy, itaconic acid ester, vinyl ether, vinyl ester, a styrene derivative, a maleic acid derivative, a fumaric acid derivative, or a cinnamic acid derivative optionally substituted by methyl, methoxy, cyano and/or by halogen;
and wherein the mesogenic radical A³ represents a radical of the formula 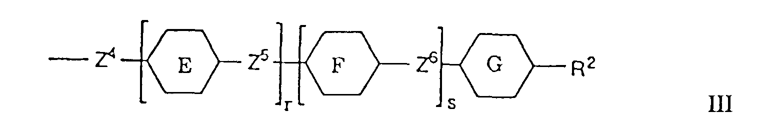 wherein
rings E and F each independently of the other represents 1,4-phenylene optionally substituted by halogen, methyl and/or by cyano, pyridine-2,5-diyl, pyrimidine-2,5-diyl, trans-1,4-cyclohexylene or trans-1,3-dioxane-2,5-diyl;
ring G represents 1,4-phenylene which is unsubstituted or mono- or di-substituted by halogen, methyl, dimethylamino, amino, nitro and/or by cyano;
Z⁴ represents a single bond, -(CH₂)ₚ-, -(CH₂)ₚO-, -O(CH₂)ₚ-, -(CH₂)ₚCOO-, -OOC(CH₂)ₚ-, -(CH₂)ₚOOC-, -COO(CH₂)ₚ-, -OOC(CH₂)ₚOOC-, -O(CH₂)ₚOOC- or -COO(CH₂)ₚO- ;
Z⁵ and Z⁶ represent a single bond, -CH₂CH₂-, -CH₂O-, -OCH₂-, -COO-, -OOC-, -(CH₂)₄-, -O(CH₂)₃- or -(CH₂)₃O-;
p is an integer from 1 to 16;
r and s are 0 or 1; and
R² represents an optically active group such as (S)- or (R)-2-butyloxy, (S)- or (R)-2-pentyloxy, (S)- or (R)-2-hexyloxy, (S)- or (R)-2-heptyloxy, (S)- or (R)-2-octyloxy, or alkyl, alkoxy or alkanoyloxy each having from 4 to 8 carbon atoms and each substituted by methyl, methoxy, cyano and/or by halogen.

2. Compounds according to claim 1, characterised in that the mesogenic radicals A¹ and A² represent a radical of the formula 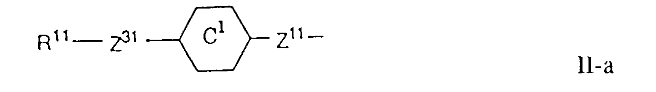 wherein
R¹¹ represents acrylate, methacrylate or epoxy;
Z³¹ represents -(CH₂)ₜ-, -(CH₂)ₜO-, -(CH₂)ₜCOO- or -(CH₂)ₜOOC-;
t is an integer from 4 to 12;
ring C¹ represents 1,4-phenylene, 2-fluoro-1,4-phenylene or 3-fluoro-1,4-phenylene; and
Z¹¹ represents -CH₂O-, -COO- or -OOC-.

3. Compounds according to claim 1, characterised in that the optically active, mesogenic radical A³ represents a radical of the formula 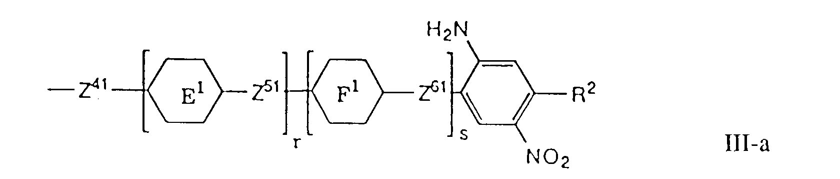 wherein
R² represents (S)- or (R)-2-butyloxy, (S)- or (R)-2-pentyloxy, (S)- or (R)-2-hexyloxy, (S)- or (R)-2-heptyloxy or (S)- or (R)-2-octyloxy;
r and s are 0 or 1;
Z⁴¹ represents -CH₂CH₂CH₂-, -OCH₂CH₂-, -CH₂CH₂O-, -COOCH₂- or -CH₂OOC-;
Z⁵¹ and Z⁶¹ represent a single bond, -CH₂CH₂-, -CH₂O-, -COO-, -OOC, -(CH₂)₄- or -(CH₂)₃O-; and
rings E¹ and F¹ represent 1,4-phenylene.

4. Compounds of the formula 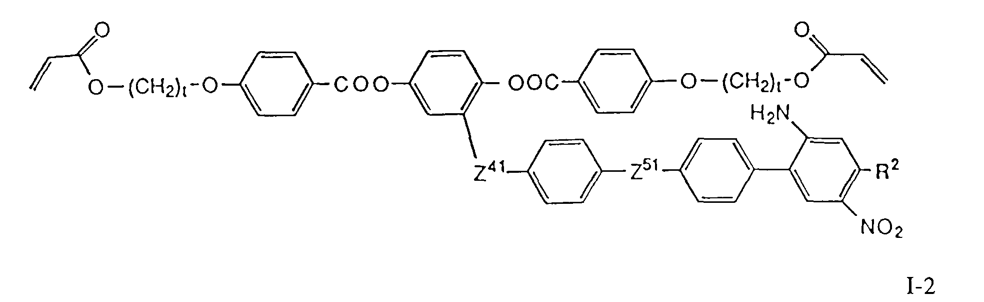 wherein
t is an integer from 4 to 12;
R² represents (S)- or (R)-2-butyloxy, (S)- or (R)-2-pentyloxy, (S)- or (R)-2-hexyloxy, (S)- or (R)-2-heptyloxy or (S)- or (R)-2-octyloxy;
Z⁴¹ represents -COOCH₂-; and
Z⁵¹ represents a single bond, -COO- or -OOC-.

5. Crosslinkable, liquid crystalline mixtures consisting of at least two components, of which at least one component is a compound of formula I defined in claim 1.

6. Crosslinkable, liquid crystalline mixtures according to claim 5, characterised in that they comprise, in addition to one or more compounds of formula I, one or more compounds from the group of compounds of the formulae 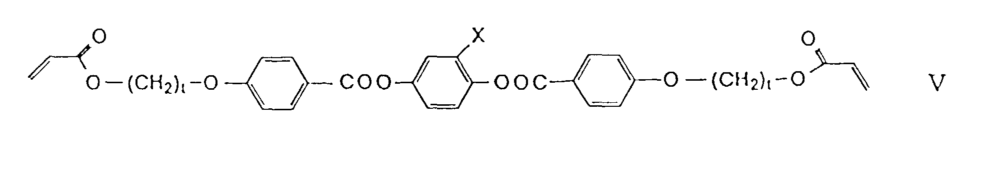 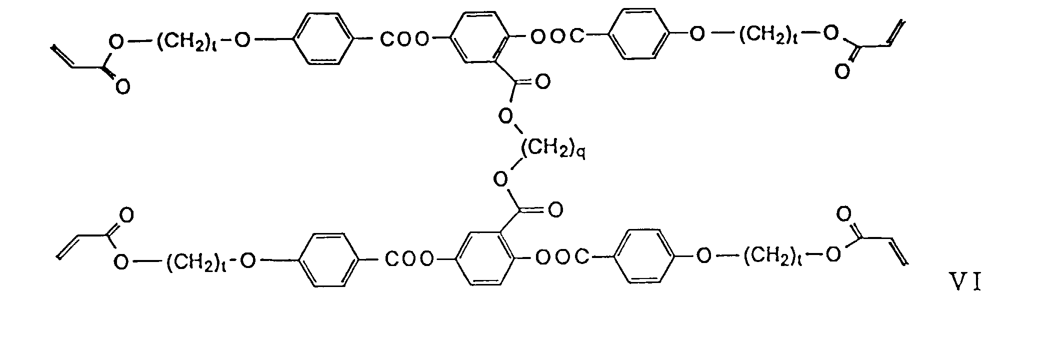 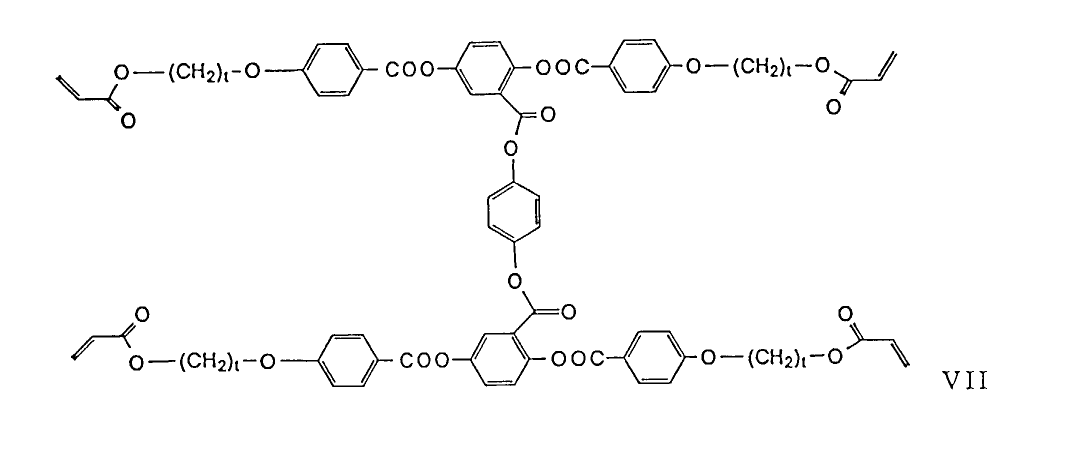 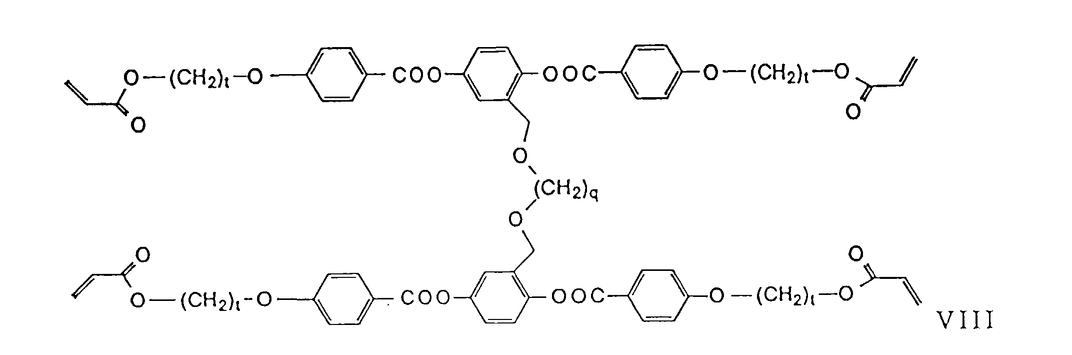 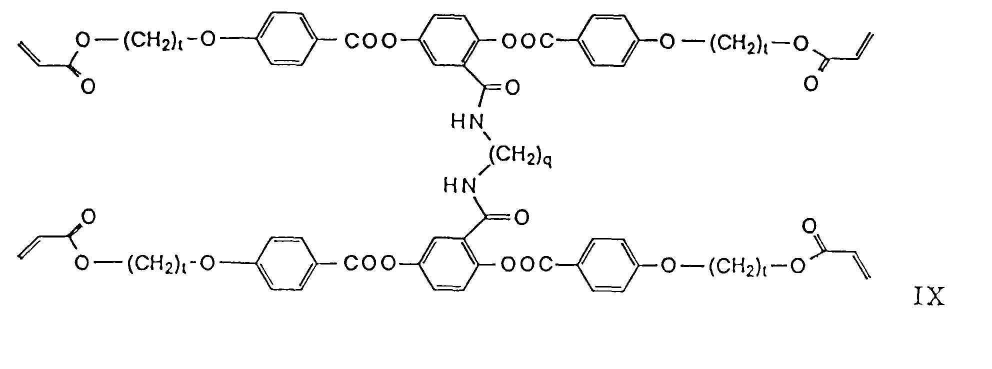 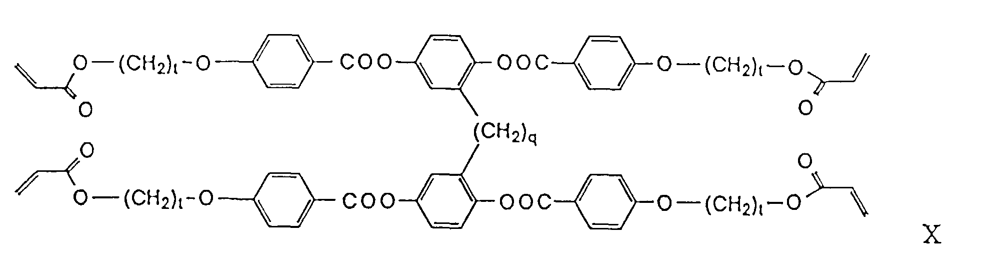 wherein t is an integer from 4 to 12 and q is an integer from 2 to 12.

7. Use of compounds according to any one of claims 1 to 4 in their crosslinked state for optical components.

8. Use of crosslinkable liquid crystalline mixtures according to claim 5 or claim 6 in their crosslinked slate for optical components.

## Revendications

1. Composés de formule générale 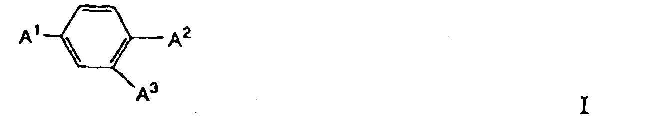 dans laquelle
A¹, A² représentent chacun un reste mésogène réticulable, et
A³ représente un reste mésogène optiquement actif, dans laquelle les groupes mésogènes A¹ et A² sont identiques et représentent des restes de formule 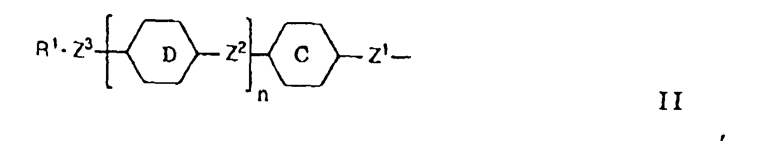
dans laquelle
les cycles C et D représentent indépendamment l'un de l'autre un groupe 1,4-phénylène, éventuellement substitué par halogène, méthyle et/ou cyano, pyridine-2,5-diyle, pyrimidine-2,5-diyle, trans-1,4-cyclohexylène ou trans-1,3-dioxane-2,5-diyle;
Z¹ représente une simple liaison, -CH₂-CH₂-, -CH₂O-, -COO-, -OOC-, -(CH₂)₄- ou -(CH₂)₃O-;
Z² représente une simple liaison, -CH₂-CH₂-, -CH₂O-, -OCH₂-, -COO-, -OOC-, -(CH₂)₄-, -O(CH₂)₃- ou -(CH₂)₃O-;
Z³ représente -(CH₂)ₘ-, -(CH₂)ₘO-, -(CH₂)ₘCOO-, ou -(CH₂)ₘOOC-;
n représente O ou 1;
m représente un nombre entier de 1 à 16; et
R¹ représente un groupe réticulable tel que : acrylate, méthacrylate, 2-chloroacrylate, 2-phénylacrylate, acryloylphénylène, acrylamide, méthacrylamide, 2-phénylacrylamide, époxy, ester d'acide itaconique, éther vinylique, ester de vinyle, dérivés du styrène, dérivés de l'acide maléique, dérivés de l'acide fumarique ou un dérivé de l'acide cinnamique éventuellement substitué par méthyle, méthoxy, cyano et/ou halogène; et dans laquelle
le reste mésogène A³ représente un reste de formule 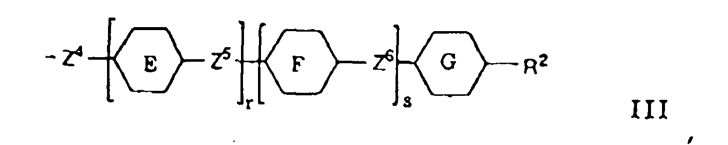 dans laquelle
les cycles E et F représentent indépendamment l'un de l'autre un groupe 1,4-phénylène, éventuellement substitué par halogène, méthyle et/ou cyano, pyridine-2,5-diyle, pyrimidine-2,5-diyle, trans-1,4-cyclohexylène ou trans-1,3-dioxane-2,5-diyle ;
le cycle G représente un groupe 1,4-phénylène non substitué ou mono- ou disubstitué par halogène, méthyle, diméthylamino, amino, nitro et/ou cyano;
Z⁴ représente une simple liaison, -(CH₂)ₚ-, -(CH₂)ₚO-, -O(CH₂)ₚ-, -(CH₂)ₚCOO-, -OOC(CH₂)ₚ-, -(CH₂)ₚOOC-, -COO(CH₂)ₚ-, -OOC(CH₂)ₚOOC-, -O(CH₂)ₚOOC- ou -COO(CH₂)ₚO-;
Z⁵, Z⁶ représentent une simple liaison, -CH₂-CH₂-, -CH₂O-, -OCH₂-, -COO-, -OOC-, -(CH₂)₄-, -O(CH₂)₃- ou -(CH₂)₃O-;
p représente un nombre entier de 1 à 16;
r, s représentent 0 ou 1; et
R² représente un groupe optiquement actif comme un groupe (S)- ou (R)-2-butyloxy, (S)- ou (R)-2-pentyloxy, (S)- ou (R)-2-hexyloxy, (S)- ou (R)-2-heptyloxy, (S)- ou (R)-2-octyloxy ou un groupe alkyle, alcoxy ou alcanoyloxy ayant de 4 à 8 atomes de carbone substitué par méthyle, méthoxy, cyano et/ou un halogène.

2. Composés selon la revendication 1, caractérisés en ce que, les restes mésogènes A¹ et A² représentent un reste de formule 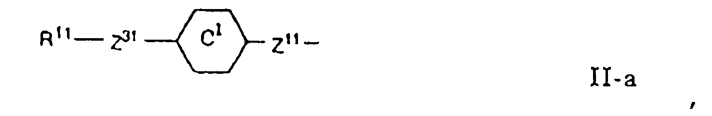 dans laquelle
R¹¹ représente acrylate, méthacrylate ou époxy;
Z³¹ représente -(CH₂)ₜ-, -(CH₂)ₜO-, -(CH₂)ₜCOO- ou -(CH₂)ₜOOC-;
t représente un nombre entier de 4 à 12;
le cycle C¹ représente 1,4-phénylène, 2-fluoro-1,4-phénylène, 3-fluoro-1,4-phénylène ; et
Z¹¹ représente un groupe -(CH₂)O-, -COO- ou -OOC-.

3. Composés selon la revendication 1, caractérisé en ce que le reste mésogène A³, optiquement actif, représente un reste de formule 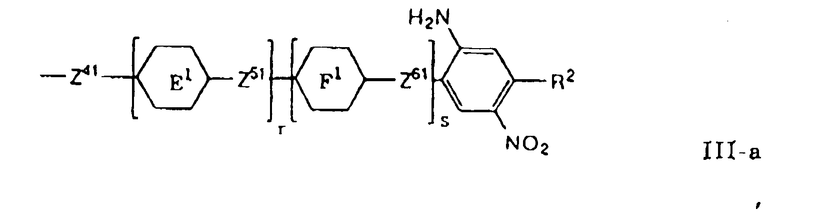 dans laquelle
R² représente un groupe (S)- ou (R)-2-butyloxy, (S)-ou (R)-2-pentyloxy, (S)- ou (R)-2-hexyloxy, (S)- ou (R)-2-heptyloxy ou (S)- ou (R)-2-octyloxy;
r, s représentent 0 ou 1;
Z⁴¹ représente -CH₂CH₂CH₂-, -OCH₂CH₂-, -CH₂CH₂O-, -COOCH₂- ou -CH₂OOC-;
Z⁵¹, Z⁶¹ représentent une simple liaison, -CH₂-CH₂-, -CH₂O- , -COO-, -OOC-, -(CH₂)₄- ou -(CH₂)₃O-; et
les cycles E¹ et F¹ représentent 1,4-phénylène.

4. Composés de formule 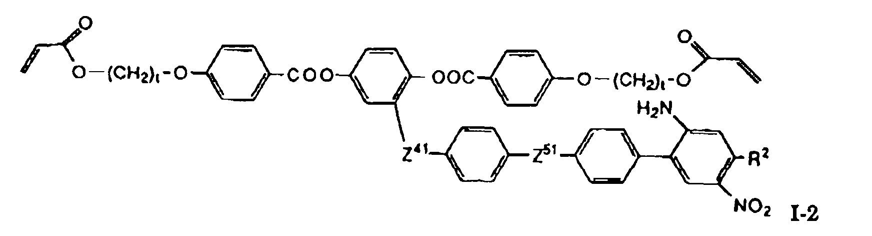 dans laquelle
t est un nombre entier de 4 à 12;
R² représente un groupe (S)- ou (R)-2-butyloxy, (S)- ou (R)-2-pentyloxy, (S)- ou (R)-2-hexyloxy, (S)- ou (R)-2-heptyloxy ou (S)- ou (R)-2-octyloxy;
Z⁴¹ représente -COOCH₂-; et
Z⁵¹ représente une simple liaison, -COO- ou -OOC-.

5. Mélanges de cristaux liquides réticulable constituée d'au moins deux composants, parmi lesquels au moins un composant est un composé de formule I définie à la revendication 1.

6. Mélanges de cristaux liquides réticulables selon la revendication 5, caractérisés en ce qu'ils contiennent en plus d'un ou de plusieurs composés de formule I, un ou plusieurs composés choisis parmi les composés du groupe de composés de formules 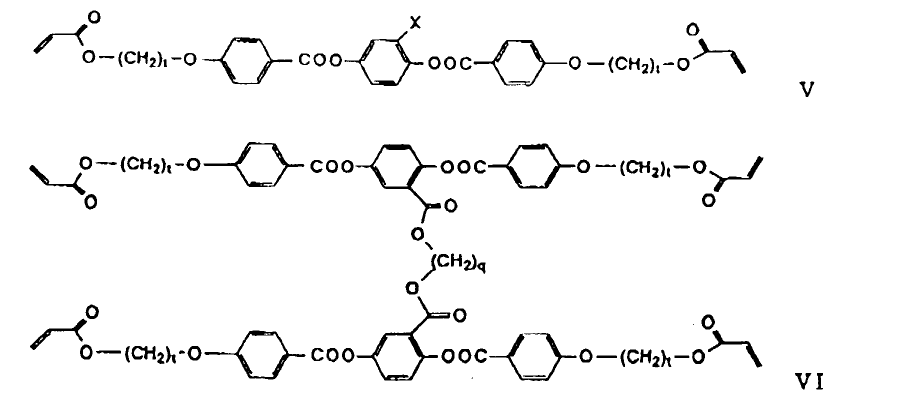 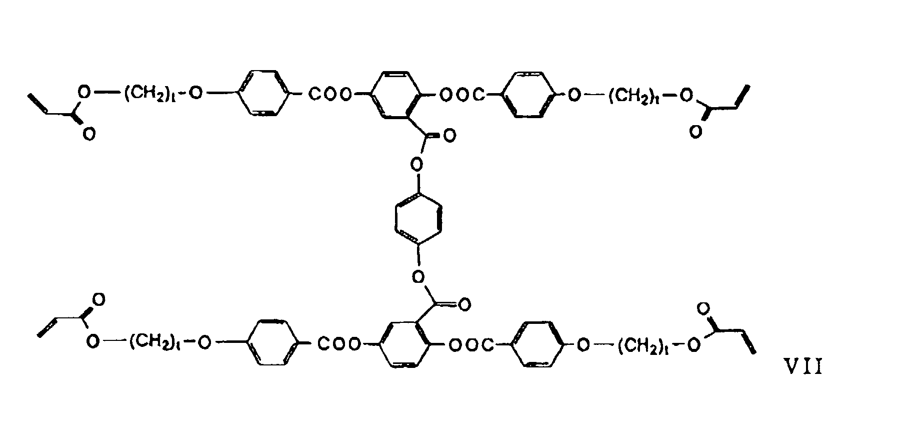 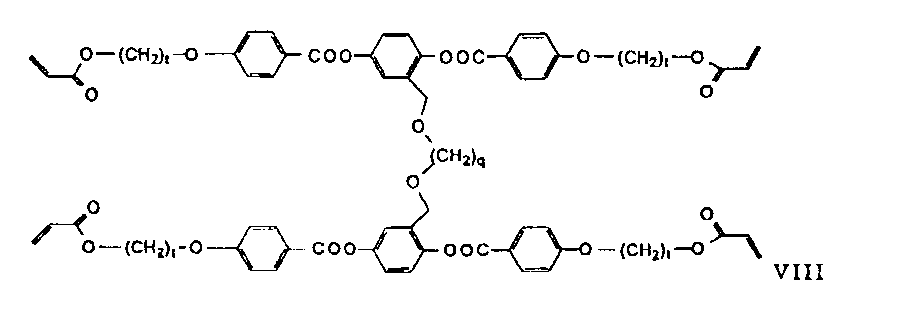 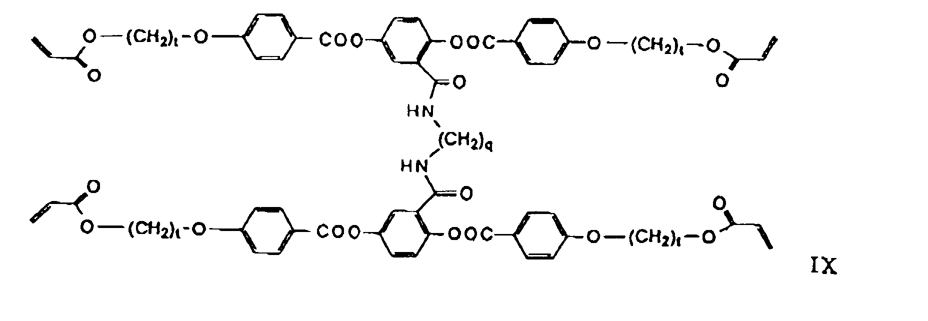 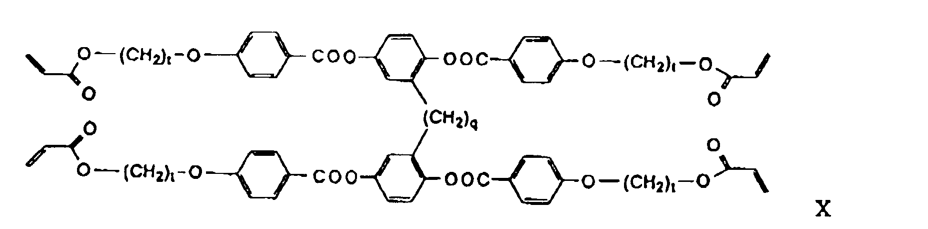 dans lesquelles t représente un nombre entier de 4 à 12 et q un nombre entier de 2 à 12.

7. Utilisation de composés selon l'une des revendications 1 à 4 à l'état réticulé pour des composants optiques.

8. Utilisation de mélanges de cristaux liquides réticulables selon la revendication 5 ou 6 à l'état réticulé pour des composants optiques.
